# EUROPÄISCHE PATENTSCHRIFT

(11) **EP 2 574 268 B1**
(45) Veröffentlichungstag und Bekanntmachung des Hinweises auf die Patenterteilung: **12.07.2017**
(21) Anmeldenummer: 11183386.9
(22) Anmeldetag: 30.09.2011
(51) Int. Cl.: A61B 1/00, A61B 1/04

(54) **Endoskop mit einstellbarer Blickrichtung**
Endoscope with adjustable view angle
Endoscope à direction d'observation réglable

(43) Veröffentlichungstag der Anmeldung: 03.04.2013
(73) Patentinhaber: Karl Storz GmbH & Co. KG, 78532 Tuttlingen (DE)
(72) Erfinder: Bürk, André, 78532 Tuttlingen (DE)

(56) Entgegenhaltungen:
- EP-A1- 1 759 629
- WO-A2-02/17773
- DE-A1- 2 328 595
- DE-A1- 19 927 816
- US-A- 3 913 568
- US-A1- 2005 177 026
- US-A1- 2005 234 296

## Beschreibung

Die vorliegende Erfindung gemäß des Anspruchs 1 bezieht sich auf ein Endoskop mit einer Blickrichtung, die innerhalb eines vorbestimmten Winkelbereichs einstellbar ist. Neben Endoskopen für medizinische und nicht-medizinische technische Anwendungen, deren Blickrichtung parallel zur Längsachse des Schafts des Endoskops ist, wurden bereits früh Endoskope mit anderen feststehenden Blickrichtungen entwickelt. Mit der Blickrichtung eines Endoskops ist hier und im Folgenden immer die Richtung vom distalen Ende des Endoskops aus gemeint, in der ein Gegenstand liegt, der in der Mitte des mittels des Endoskops erfassten Bilds erscheint. Bei vielen Anwendungen ist jedoch eine feste Blickrichtung nachteilig. Im ungünstigsten Fall muss beispielsweise während eines medizinischen Eingriffs mehrfach das Endoskop gewechselt werden. In solchen Fällen ist die Verwendung eines Endoskops mit einer in situ einstellbaren bzw. verstellbaren Blickrichtung vorteilhaft. Dokument EP 1759629 A1 offenbart ein Endoskop mit einem distalem Kopf, enthaltend ein optisches Bildgebungssystem mit Kamera, wobei der distale Kopf zur Einstellung der Blickrichtung um zwei parallele Achsen einer Gelenkanordnung verschwenkbar gelagert ist.

Dokument US 2005/177026 A1 offenbart ein Endoskop mit einem distalen Prisma, das zur Einstellung der Blickrichtung um zwei orthogonale Achsen drehbar gelagert ist.

Dokumente DE 2328595 A1, WO 02/17773 A2 und DE 19927816 A1 offenbaren Endoskope mit jeweils einem distalen Prisma oder Spiegel, die zur Einstellung der Blickrichtung um eine Achse drehbar gelagert sind.

Zum Einstellen bzw. Verstellen der Blickrichtung kann am distalen Ende des Endoskops eine bewegbare Lichtlenkeinrichtung vorgesehen sein, von deren Orientierung die Blickrichtung des Endoskops abhängt. Die Lichtlenkeinrichtung umfasst beispielsweise ein Prisma oder einen Spiegel, dessen Orientierung bestimmt, in welcher Richtung (bezogen auf das distale Ende des Endoskops) beobachtbare Gegenstände liegen bzw. aus welcher Richtung auf das distale Ende des Endoskops fallendes Licht in einen Beobachtungsstrahlengang eingekoppelt und/oder auf einen lichtempfindlichen Bildsensor gelenkt wird.

Bei der Konstruktion eines Endoskops mit einstellbarer Blickrichtung ist eine ganze Reihe von Anforderungen zu berücksichtigen. Insbesondere ist zur Erzielung einer hohen Lichtstärke eine Lichtlenkeinrichtung mit einem möglichst großen Querschnitt erwünscht. Ferner ist ein möglichst großer Winkelbereich, innerhalb dessen die Blickrichtung einstellbar ist, wünschenswert. Gleichzeitig steht am distalen Ende eines Endoskops immer nur ein begrenzter Bauraum für die Lichtlenkeinrichtung und ihre Bewegbarkeit zur Verfügung. Mit zunehmender Miniaturisierung bzw. abnehmendem Schaftquerschnitt wird der zur Verfügung stehende Bauraum immer kleiner. Es sind deshalb neue Ansätze erforderlich, um die für die Konstruktion eines Endoskops geltenden Anforderungen in höherem Maße zu erfüllen.

Eine Aufgabe der vorliegenden Erfindung besteht darin, ein verbessertes Endoskop mit einer Blickrichtung, die innerhalb eines vorbestimmten Winkelbereichs einstellbar ist, zu schaffen.

Diese Aufgabe wird durch den Gegenstand des unabhängigen Anspruchs 1 gelöst. Weiterbildungen sind in den abhängigen Ansprüchen angegeben.

Ein Endoskop mit einer Blickrichtung, die innerhalb eines vorbestimmten Winkelbereichs einstellbar ist, umfasst eine bewegbare Lichtlenkeinrichtung am distalen Ende des Endoskops, von deren Orientierung die Blickrichtung des Endoskops abhängt, und eine Gelenkeinrichtung zum bewegbaren Halten der Lichtlenkeinrichtung, wobei die Gelenkeinrichtung für eine Bewegbarkeit der Lichtlenkeinrichtung ausgebildet ist, die mehr als nur eine Schwenkbarkeit um eine einzige Schwenkachse umfasst.

Die Blickrichtung des Endoskops ist die Richtung - bezogen auf das distale Ende des Endoskops - in der ein Gegenstand liegt, der in der Mitte eines mittels des Endoskops erfassten Bilds erscheint. Die Lichtlenkeinrichtung umfasst erfindungsgemäß ein Prisma oder einen Spiegel und definiert durch ihre Orientierung bzw. ihre Winkelposition die Blickrichtung des Endoskops. Zusätzlich kann die Blickrichtung durch eine translatorische Bewegung der Lichtlenkeinrichtung beeinflussbar sein.

Die Bewegbarkeit der Lichtlenkeinrichtung umfasst erfindungsgemäß eine Abfolge von Schwenkbewegungen um verschiedene Schwenkachsen. Dabei schwenkt die Lichtlenkeinrichtung jederzeit nur um eine Schwenkachse oder teilweise oder jederzeit gleichzeitig um mehrere Schwenkachsen, die erfindungsgemäß parallel zu einander sind.

Die Lichtlenkeinrichtung kann ausgebildet sein, um bei einem Schwenken der Lichtlenkeinrichtung um einen bestimmten Winkel ein Schwenken der Blickrichtung um den gleichen Winkel zu bewirken. Beispielsweise ist die Lichtlenkeinrichtung ein Objektiv oder eine Linse, mit dem bzw. der ein in der Bildebene des Objektivs bzw. der Linse angeordneter lichtempfindlicher Bildsensor starr verbunden ist, der mit der Lichtlenkeinrichtung bewegbar ist. Alternativ kann die Lichtlenkeinrichtung ausgebildet sein, um bei einem Schwenken der Lichtlenkeinrichtung um einen Winkel ein Schwenken der Blickrichtung um einen doppelt so großen Winkel zu bewirken. Ein Beispiel ist ein Spiegel oder ein Prisma mit einer reflektierenden Fläche, das Licht von beobachteten Gegenständen unmittelbar oder mittelbar zu einer Kamera oder einem lichtempfindlichen Bildsensor oder zu einem Okular lenkt.

Ein lichtempfindlicher Bildsensor kann unmittelbar lichtstromabwärts der Lichtlenkeinrichtung oder eines lichtstromabwärts der Lichtlenkeinrichtung angeordneten Objektivs am distalen Ende des Endoskops angeordnet sein. Das Endoskop kann eine Anordnung von Stablinsen oder ein geordnetes Bündel von Lichtleitern umfassen, um das von der Lichtlenkeinrichtung gelenkte Licht zu einem Okular, einem lichtempfindlichen Bildsensor oder zu einer Kamera am proximalen Ende des Endoskops zu leiten.

Eine Gelenkeinrichtung, die für eine Bewegbarkeit der Lichtlenkeinrichtung ausgebildet ist, die erfindungsgemäß mehr als nur eine Schwenkbarkeit um eine einzige Schwenkachse umfasst, kann bei gegebenem Bauraum eine größere Lichtlenkeinrichtung, insbesondere einen größeren Querschnitt der Lichtlenkeinrichtung ermöglichen, insbesondere eine größere Länge und/oder eine größere Höhe eines Prismas. Damit kann beispielsweise ein größerer Winkelbereich von möglichen Blickrichtungen und/oder eine größere Lichtstärke ermöglicht werden. Insbesondere kann eine erfindungsgemäße Gelenkeinrichtung, die für eine Bewegung der Lichtlenkeinrichtung ausgebildet ist, die mehr als nur ein Schwenken um eine einzige Schwenkachse umfasst, eine bei mehreren Blickrichtungen des Endoskops hinsichtlich des für die Lichtlenkeinrichtung erforderlichen Bauraums und hinsichtlich der Anordnung der Lichtlenkeinrichtung innerhalb des Beobachtungsstrahlengangs optimale Position der Lichtlenkeinrichtung ermöglichen.

Bei einem Endoskop, wie es hier beschrieben ist, umfasst die Gelenkeinrichtung erfindungsgemäß ein erstes Gelenk, das eine erste Schwenkachse definiert, und ein zweites Gelenk, das eine zweite Schwenkachse definiert.

Die erste Schwenkachse und die zweite Schwenkachse sind voneinander verschieden und insbesondere parallel und insbesondere senkrecht zur Längsachse des Endoskops und zu den einstellbaren Blickrichtungen. Die Gelenkeinrichtung kann für ein gleichzeitiges oder alternatives Schwenken um die erste Schwenkachse und die zweite Schwenkachse ausgebildet sein. Gelenke, die jeweils ein Schwenken um eine zugeordnete Schwenkachse ermöglichen, können besonders spiel- und reibungsarm ausgebildet sein und damit eine präzise Bewegung der Lichtlenkeinrichtung ermöglichen.

Insbesondere sind das erste Gelenk ausgebildet und angeordnet, um eine Schwenkbewegung der Lichtlenkeinrichtung um die erste Schwenkachse zu ermöglichen, wenn die Blickrichtung in einem ersten Teilbereich des vorbestimmten Winkelbereichs liegt, und das zweite Gelenk ausgebildet und angeordnet, um eine Schwenkbewegung der Lichtlenkeinrichtung um die zwei Schwenkachse zu ermöglichen, wenn die Blickrichtung in einem zweiten Teilbereich des vorbestimmten Winkelbereichs liegt.

Insbesondere ist die Gelenkeinrichtung ausgebildet, um eine Schwenkbewegung der Lichtlenkeinrichtung um die erste Schwenkachse nur dann zu ermöglichen, wenn die Blickrichtung in dem ersten Teilbereich liegt, und/oder um eine Schwenkbewegung der Lichtlenkeinrichtung um die zweite Schwenkachse nur dann zu ermöglichen, wenn die Blickrichtung in dem zweiten Teilbereich liegt.

Insbesondere grenzen der erste Teilbereich und der zweite Teilbereich aneinander an.

Der erste Teilbereich und der zweite Teilbereich grenzen aneinander an, indem die Obergrenze des ersten Teilbereichs der Untergrenze des zweiten Teilbereichs entspricht oder umgekehrt. Beide Teilbereiche weisen also insbesondere - von mechanischem Spiel abgesehen - keinen Überlapp auf.

Ein Endoskop mit zwei Schwenkachsen, wie es hier beschrieben ist, umfasst insbesondere ferner eine Einrichtung zum Hemmen einer Schwenkbewegung um die erste Schwenkachse, wobei die Einrichtung zum Hemmen ausgebildet ist, um die Schwenkbewegung zumindest dann zu hemmen, wenn die Blickrichtung außerhalb des ersten Teilbereichs liegt.

Die Einrichtung zum Hemmen kann ausgebildet sein, um die Schwenkbewegung um die erste Schwenkachse innerhalb des gesamten vorbestimmten Winkelbereichs oder nur innerhalb eines Teils des vorbestimmten Winkelbereichs, insbesondere nur außerhalb des ersten Teilbereichs zu hemmen. Die Hemmung der Schwenkbewegung um die erste Schwenkachse kann bewirken, dass die Lichtlenkeinrichtung bevorzugt um die zweite Schwenkachse schwenkt, wenn dies möglich ist. Dadurch kann auf einfache Weise eine automatische Auswahl der Schwenkachse erfolgen.

Ein Endoskop mit zwei Schwenkachsen, wie es hier beschrieben ist, kann ferner eine Einrichtung zum Blockieren einer Schwenkbewegung um die zweite Schwenkachse, wobei die Einrichtung zum Blockieren ausgebildet ist, um die Schwenkbewegung um die zweite Schwenkachse zu blockieren, wenn die Blickrichtung nicht innerhalb des zweiten Teilbereichs liegt, umfassen.

Die Einrichtung zum Blockieren umfasst beispielsweise einen Riegel, der in einer vorbestimmten Winkelposition so eingreift, dass eine Schwenkbewegung um die zweite Schwenkachse blockiert ist. Insbesondere zusammen mit einer Einrichtung zum Hemmen einer Schwenkbewegung um die erste Schwenkachse, wie sie oben beschrieben ist, kann die Einrichtung zum Blockieren eine automatische bzw. selbsttätige Auswahl der Schwenkachse abhängig von der Blickrichtung ermöglichen.

Ein Endoskop mit zwei Schwenkachsen, wie es hier beschrieben ist, kann ferner ein elastisches Element umfassen, das so mit der Gelenkeinrichtung gekoppelt ist, dass zumindest entweder das erste Gelenk in Richtung zu einer ersten vorbestimmten Winkelposition vorgespannt ist oder das zweite Gelenk in Richtung zu einer zweiten vorbestimmten Winkelposition vorgespannt ist.

Das elastische Element umfasst beispielsweise eine Blattfeder, eine Spiralfeder oder ein Bauteil aus einem Elastomer. Insbesondere sind das erste Gelenk und/oder das zweite Gelenk in Richtung zu den Winkelpositionen vorgespannt, die sie an der Grenze zwischen den Teilbereichen des vorbestimmten Winkelbereichs einnehmen. Eine Vorspannung des ersten Gelenks und/oder des zweiten Gelenks kann auf einfache Weise eine selbsttätige Auswahl der Schwenkachse abhängig von der eingestellten Blickrichtung des Endoskops ermöglichen.

Bei einem Endoskop mit zwei Schwenkachsen, wie es hier beschrieben ist, kann die Gelenkeinrichtung einen ersten Lenker, der das erste Gelenk und das zweite Gelenk verbindet, und einen zweiten Lenker, der ein drittes Gelenk und ein viertes Gelenk verbindet, umfassen, wobei das zweite Gelenk und das vierte Gelenk mit der Lichtlenkeinrichtung mechanisch starr verbunden sind.

Um eine Bewegung der Lichtlenkeinrichtung zu ermöglichen, die mehr als nur eine Parallelverschiebung entlang eines gekrümmten Pfads darstellt, sind das erste Gelenk, das zweite Gelenk, das dritte Gelenk und das vierte Gelenk nicht in einem Parallelogramm angeordnet. Dazu sind insbesondere der Abstand des ersten Gelenks und des dritten Gelenks und der Abstand des zweiten Gelenks und des vierten Gelenks unterschiedlich und/oder die Länge des ersten Lenkers bzw. der Abstand des ersten Gelenks und des zweiten Gelenks einerseits und die Länge des zweiten Lenkers bzw. der Abstand des dritten Gelenks und des vierten Gelenks andererseits unterschiedlich.

Bei einer insbesondere hinsichtlich mechanischer Robustheit vorteilhaften zumindest teilweise symmetrischen Anordnung der Gelenkeinrichtung an zwei gegenüberliegenden Seiten der Lichtlenkeinrichtung sind insbesondere je zwei parallele erste Lenker an gegenüberliegenden Seiten der Lichtlenkeinrichtung und/oder zwei parallele zweite Lenker an gegenüberliegenden Seiten der Lichtlenkeinrichtung angeordnet.

Die Verwendung zweier nicht gleich langer und/oder nicht paralleler Lenker kann bei geeigneter Ausgestaltung eine Bewegung der Lichtlenkeinrichtung ermöglichen, die aus einer von der Blickrichtung abhängigen Überlagerung einer Schwenkbewegung und einer Translationsbewegung besteht. Diese Bewegung kann eine hinsichtlich des erforderlichen Bauraums und hinsichtlich des Beobachtungsstrahlengangs optimale Anordnung der Lichtlenkeinrichtung bei mehreren Blickrichtungen, insbesondere bei extremen Blickrichtungen am Rand des vorbestimmten Winkelbereichs ermöglichen.

Bei einem Endoskop, wie es hier beschrieben ist, kann die Gelenkeinrichtung eine Gleitflächeneinrichtung zum Führen entlang eines Pfads umfassen.

Die Gleitflächeneinrichtung kann zum Führen entlang eines geraden oder gekrümmten Pfads ausgebildet sein. Die Gelenkeinrichtung kann mehrere Gleitflächeneinrichtungen zum Führen entlang je eines geraden oder gekrümmten Pfads umfassen, wobei beispielsweise das proximale Ende und das distale Ende eines Prismas durch je eine zugeordnete Gleitflächeneinrichtung geführt wird.

Ferner kann die Gelenkeinrichtung ein Gelenk zum Schwenken um eine Achse und eine Gleitflächeneinrichtung zum Führen entlang eines Pfads umfassen. Insbesondere sind das Gelenk und die Linearführung so angeordnet, dass die Linearführung ein Verfahren des Gelenks und der Lichtlenkeinrichtung entlang des durch die Linearführung vorbestimmten Pfads ermöglicht. Alternativ können das Gelenk und die Linearführung so angeordnet sein, dass das Gelenk ein Schwenken der Linearführung, des durch die Linearführung definierten Pfads und der Lichtlenkeinrichtung um eine durch das Gelenk definierte Schwenkachse ermöglicht.

Eine Gleitflächeneinrichtung mit nahezu beliebig formbaren Gleitflächen kann eine nahezu beliebig komplexe Bewegung der Lichtlenkeinrichtung ermöglichen. Damit kann unter Umständen für jede einstellbare Blickrichtung des Endoskops eine optimale Anordnung und Ausrichtung bzw. Orientierung der Lichtlenkeinrichtung realisierbar sein.

Bei einem Endoskop, bei dem die Gelenkeinrichtung eine Gleitflächeneinrichtung und ein Gelenk umfasst, kann die Gelenkeinrichtung ferner eine Kopplungseinrichtung umfassen, die ein Schwenken um die durch das Gelenk definierte Schwenkachse und eine Bewegung entlang dem durch die Linearführung definierten Pfad miteinander koppelt.

Die Kopplungseinrichtung umfasst insbesondere einen oder mehrere Lenker, eine Kulissenführung, eine Nockensteuerung, ein oder mehrere Zahnräder oder eine Kombination derselben. Die Kopplungseinrichtung kann es ermöglichen, dass trotz mehrerer Freiheitsgrade der Lichtlenkeinrichtung nur ein Steuerdraht, eine Benutzerschnittstelle, ein Motor oder eine andere Steuereinrichtung erforderlich ist, um eine vorbestimmte Bewegung der Lichtlenkeinrichtung zu ermöglichen.

Bei einem Endoskop, wie es hier beschrieben ist, kann die Gelenkeinrichtung ferner zumindest entweder eine Kulissenführung oder einen Lenker oder eine Nockensteuerung oder ein elastisches Element umfassen.

Bei allen hier dargestellten Endoskopen können beispielsweise mehrere Steuerdrähte und/oder mehrere Motoren oder ein Zahnradgetriebe oder ein anderes Getriebe oder eine andere Kopplungseinrichtung am proximalen Ende vorgesehen sein, um durch die Gelenkeinrichtung bereitgestellte Freiheitsgrade unabhängig voneinander oder synchronisiert zu steuern.

Bei einem Endoskop, wie es hier beschrieben ist, kann die Lichtlenkeinrichtung zumindest entweder ein Prisma, ein Objektiv, eine Linse oder einen Spiegel umfassen.

Ein Endoskop, wie es hier beschrieben ist, kann ferner einen lichtempfindlichen Bildsensor umfassen, der so mit der Lichtlenkeinrichtung gekoppelt ist, dass er mit der Lichtlenkeinrichtung bewegbar ist.

Der lichtempfindliche Bildsensor kann mit der Lichtlenkeinrichtung mechanisch starr verbunden sein. Alternativ kann der lichtempfindliche Bildsensor so mit der Lichtlenkeinrichtung gekoppelt sein, dass er synchron mit dieser bewegbar, jedoch beispielsweise entlang einem anderen Pfad verschiebbar oder um eine andere Achse schwenkbar ist.

### Kurzbeschreibung der Figuren

Nachfolgend werden Ausführungsformen anhand der beigefügten Figuren näher erläutert. Es zeigen:
- Figur 1: eine schematische Darstellung eines Endoskops mit einer einstellbaren Blickrichtung;
- Figur 2: eine schematische Darstellung eines distalen Endes eines Endoskops;
- Figur 3: eine weitere schematische Darstellung des distalen Endes aus Figur 2;
- Figur 4: eine schematische Darstellung eines distalen Endes eines Endoskops;
- Figur 5: eine weitere schematische Darstellung des distalen Endes aus Figur 4;
- Figur 6: eine weitere schematische Darstellung des distalen Endes aus den Figuren 4 und 5;
- Figur 7: eine schematische Darstellung eines distalen Endes eines Endoskops;
- Figur 8: eine weitere schematische Darstellung des distalen Endes aus Figur 7;
- Figur 9: eine weitere schematische Darstellung des distalen Endes aus den Figuren 7 und 8;
- Figur 10: eine schematische Darstellung eines weiteren distalen Endes eines Endoskops;
- Figur 11: eine weitere schematische Darstellung des distalen Endes aus Figur 10.

### Beschreibung der Ausführungsformen

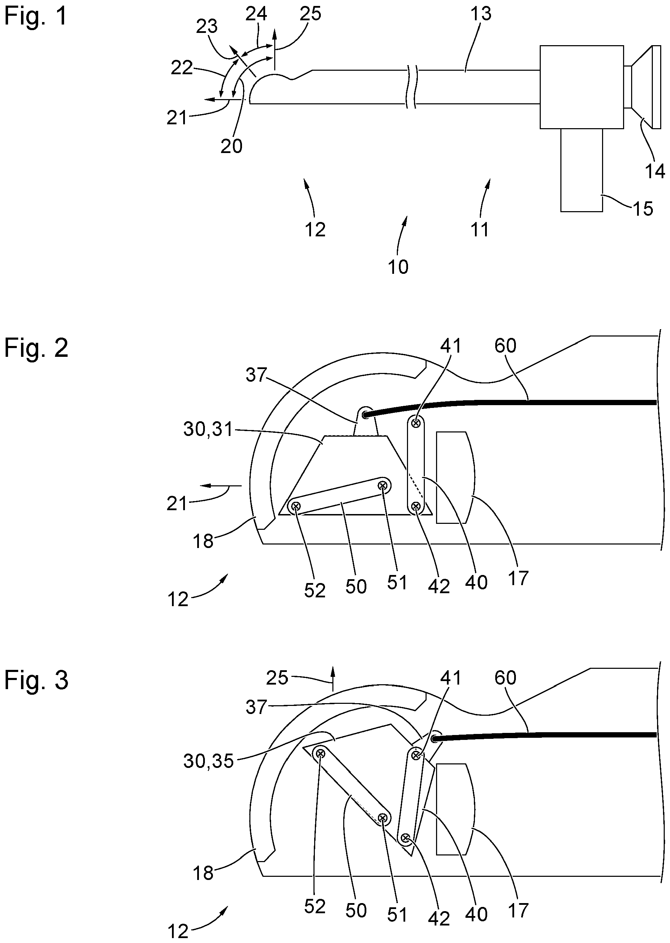

Figur 1 zeigt eine schematische Darstellung eines Endoskops 10 mit einem proximalen Ende 11 und einem distalen Ende 12. Das Endoskop 10 weist einen Schaft 13 auf, der sich vom proximalen Ende 11 bis zum distalen Ende 12 erstreckt. Der Schaft 13 kann jeweils teilweise oder vollständig starr oder flexibel, gerade oder gekrümmt sein. Am proximalen Ende 11 weist das Endoskop 10 ein Okular 14 zur unmittelbaren visuellen Betrachtung durch das menschliche Auge und/oder eine Kupplung für eine Kamera auf. Ferner weist das Endoskop 10 am proximalen Ende 11 eine Kupplung 15 zur Kopplung des Endoskops 10 mit einer externen Lichtquelle mittels eines Lichtleitkabels auf.

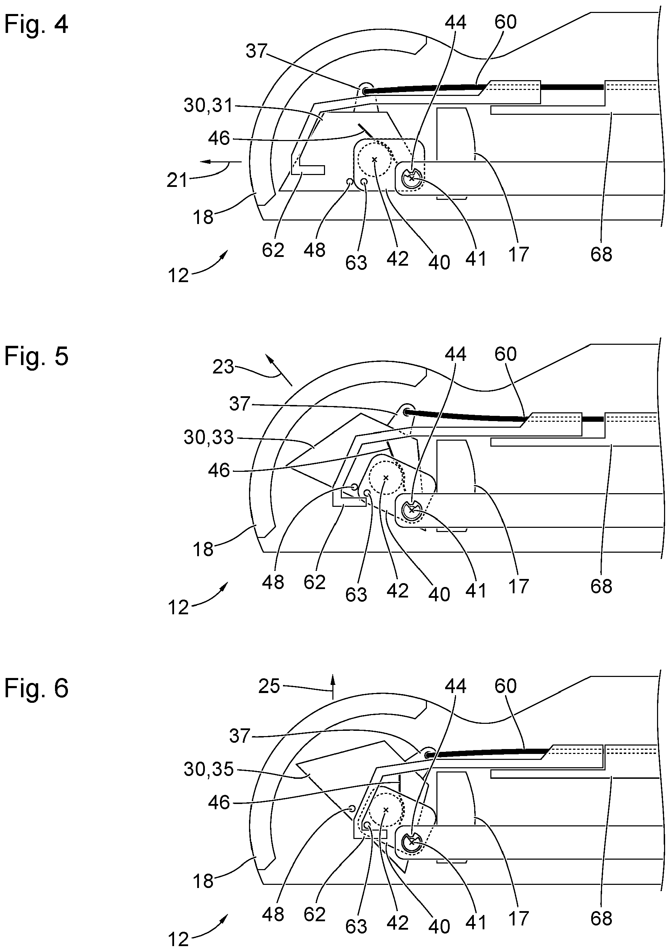

Am distalen Ende 12 ist eine in Figur 1 nicht dargestellte bewegbare Lichtlenkeinrichtung vorgesehen, die eine insbesondere kontinuierlich einstellbare Blickrichtung 21, 23, 25 ermöglicht. Zwischen extremen Blickrichtungen 21, 25 liegt ein Winkelbereich 20, innerhalb dessen die Blickrichtung einstellbar ist. In Figur 1 sind zwei Teilbereiche 22, 24 des Winkelbereichs 20 angedeutet, auf die bei dem unten anhand der Figuren 4 bis 6 dargestellten Ausführungsbeispiel Bezug genommen wird. Der erste Teilbereich 22 reicht von einer ersten Blickrichtung 21 im Wesentlichen parallel zur Längsachse des Schafts 13 bis zu einer zweiten Blickrichtung 23. Der zweite Teilbereich 24 reicht von der zweiten Blickrichtung 23 bis zu einer dritten Blickrichtung 25.

Die Figuren 2 bis 8 zeigen schematische Darstellungen von Ausführungsbeispielen des distalen Endes 12 des Endoskops 10 aus Figur 1. Die Darstellung in den Figuren 2 bis 8 weisen insofern den Charakter von Schnittdarstellungen auf, als sie das Innere des Schafts 13 am distalen Ende 12 des Endoskops 10 zeigen. Die Zeichenebenen der Figuren 2 bis 8 sind parallel zur Zeichenebene der Figur 1, zur Längsachse des Schafts 13 und zu den einstellbaren Blickrichtungen 21, 23, 25 des Endoskops 10.

Der Aufbau des Schafts 13 des Endoskops 10, insbesondere die Struktur seiner Wand und/oder seine Wände ist in den Figuren 2 bis 8 nicht dargestellt. In den Figuren 2 bis 8 sind jeweils eine distale Linse 17 und ein Fensterbauteil 18 angedeutet, die den für eine bewegbare Lichtlenkeinrichtung 30 zur Verfügung stehenden Bauraum begrenzen. Durch das Fensterbauteil 18 in das distale Ende 12 des Endoskops 10 eintretendes Licht von einem Objekt kann durch die Lichtlenkeinrichtung 30 zur distalen Linse 17 gelenkt werden. Lichtstromabwärts der Linse 17 kann eine Stablinsenoptik oder ein lichtempfindlicher Bildsensor angeordnet sein. Das Fensterbauteil 18, die Lichtlenkeinrichtung 30 und die distale Linse 17 deuten den Beobachtungsstrahlengang des Endoskops 10 an.

Bei dem in den Figuren 2 bis 8 dargestellten Ausführungsbeispielen ist die Lichtlenkeinrichtung 30 ein Prisma mit einer dem Fensterbauteil 18 zugewandten Lichteintrittsseite, einer der distalen Linse 17 zugewandten Lichtaustrittsseite und einer reflektierenden, insbesondere totalreflektierenden ebenen Fläche, die sich von der Lichteintrittsseite bis zur Lichtaustrittsseite erstreckt.

Die Figuren 2 und 3 zeigen schematische Darstellungen eines Ausführungsbeispiels eines distalen Endes 12 eines Endoskops, bei dem das Prisma 30 mittels einer Gelenkeinrichtung aus einem ersten Lenker 40 und einem zweiten Lenker 50 bewegbar im Endoskop gelagert ist. Parallel zu den Lenkern 40, 50 können weitere Lenker an einer gegenüberliegenden, vom Betrachter abgewandten Seite des Prismas 30 angeordnet sein. Der erste Lenker 40 ist über ein erstes Gelenk 41 mit dem Schaft 13 des Endoskops 10 und über ein zweites Gelenk 42 mit dem Prisma 30 gelenkig verbunden. Der zweite Lenker 50 ist über ein drittes Gelenk 51 mit dem Schaft 13 und über ein viertes Gelenk 52 mit dem Prisma 30 verbunden. Das erste Gelenk 41 und das dritte Gelenk 51 sind ortsfest im Schaft 13 des Endoskops verankert. Das zweite Gelenk 42 und das vierte Gelenk 52 sind unmittelbar oder mittelbar an voneinander beabstandeten Orten am Prisma 30 befestigt. Das erste Gelenk 41, das zweite Gelenk 42, das dritte Gelenk 51 und das vierte Gelenk 52 definieren jeweils - ggf. zusammen mit entsprechenden weiteren Gelenken paralleler und in den Figuren 2 und 3 nicht dargestellter Lenker - eine Schwenkachse senkrecht zu den Zeichenebenen der Figuren 2 und 3.

Die Figuren 2 und 3 zeigen das Prisma in zwei verschiedenen Positionen 31, 35, die der ersten Blickrichtung 21 bzw. der dritten Blickrichtung 25 (vgl. Figur 1) entsprechen bzw. zuzuordnen sind. Zum Bewegen bzw. zum mechanischen Steuern des Prismas 30 ist ein Steuerdraht 60 vorgesehen, dessen distales Ende gelenkig mit einem Steuerhebel 37 am Prisma 30 verbunden ist. Der Steuerdraht 60 erstreckt sich insbesondere vom proximalen Ende 11 bis zum distalen Ende 12 des Endoskops 10 (vgl. Figur 1). Am proximalen Ende 11 des Endoskops ist der Steuerdraht 60 insbesondere mit einem Schieber, einem Drehrad oder einer anderen Benutzerschnittstelle gekoppelt, an der medizinisches Personal die Blickrichtung des Endoskops 10 einstellen kann. Alternativ kann der Steuerdraht 60 am proximalen Ende 11 des Endoskops 10 mit einem Motor zur motorischen Verstellung der Blickrichtung gekoppelt sein.

Bei dem Ausführungsbeispiel der Figuren 2 und 3 sind die durch das dritte Gelenk 51 definierte Schwenkachse möglichst nah am Krümmungsmittelpunkt der inneren Oberfläche des Fensterbauteils 18 angeordnet. Die durch das vierte Gelenk 52 definierte Schwenkachse des zweiten Lenkers 50 ist möglichst nah an dem Punkt des Prismas 30 angeordnet, der dem Fensterbauteil 18 am nächsten kommt. Der erste Lenker 40, das erste Gelenk 41 und das zweite Gelenk 42 sind so ausgebildet und angeordnet, dass die der distalen Linse 17 zugewandte Seite des Prismas 30 über einen möglichst großen Schwenkbereich möglichst optimal relativ zur distalen Linse 17 positioniert ist. Durch eine Anordnung und Ausbildung der Lenker 40, 50 und der Gelenke 41, 42, 51, 52 abweichend von den Darstellungen in den Figuren 2 und 3 kann die Bewegung des Prismas 30 in weiten Grenzen an die aus dem Beobachtungsstrahlengang, den optischen Eigenschaften des Prismas 30 und dem für dieses bereitstehenden Bauraum angepasst werden.

Die Figuren 4 bis 6 zeigen ein Ausführungsbeispiel eines distalen Endes 12 eines Endoskops, das in einigen Merkmalen dem Ausführungsbeispiel der Figuren 2 und 3 ähnelt, insbesondere in der Ausbildung der Lichtlenkeinrichtung 30 als Prisma, in der Begrenzung des für das Prisma 30 zur Verfügung stehenden Bauraums durch eine distale Linse 17 und ein Fensterbauteil 18 sowie in der mechanischen Steuerung des Prismas 30 mittels eines Steuerdrahts 60, dessen distales Ende gelenkig mit einem Steuerhebel 37 am Prisma 30 gekoppelt ist.

Bei dem Ausführungsbeispiel der Figuren 4 bis 6 ist das Prisma 30 mittels eines Lenkers 40 im Schaft 13 des Endoskops gelagert. Der Lenker 40 ist bei dem Ausführungsbeispiel der Figuren 4 bis 6 als rechteckige Platte mit abgerundeten Ecken und im Wesentlichen parallel zu den Zeichenebenen der Figuren 4 bis 6 dargestellt. Der Lenker 40 ist über ein erstes Gelenk 41 mit dem Endoskop bzw. dessen Schaft 13 und über ein zweites Gelenk 42 mit dem Prisma 30 verbunden. Die durch das erste Gelenk 41 bzw. das zweite Gelenk 42 definierten Schwenkachsen sind parallel zueinander und senkrecht zu den Zeichenebenen der Figuren 4 bis 6. Parallel zum Lenker 40 kann ein weiterer, in den Figuren 4 bis 6 nicht dargestellter Lenker an einer vom Betrachter abgewandten Seite des Prismas 30 vorgesehen sein.

Am ersten Gelenk 41 ist ein Vorsprung 44 am feststehenden Teil des Gelenks 41 vorgesehen, der in radialer Richtung in eine Ausnehmung in einem Achszapfen am Lenker 40 eingreift, wobei der Vorsprung 44 in umfänglicher Richtung kleiner ist als die Ausnehmung. Dadurch begrenzt der Vorsprung 44 die Schwenkbewegung des Lenkers 40 auf eine Schwenkbewegung zwischen den beiden in den Figuren 4 und 5 dargestellten Winkelpositionen. Alternativ kann abweichend von der Darstellung in den Figuren 4 bis 6 ein die Schwenkbewegung des Lenkers 40 begrenzender mechanischer Anschlag an einer anderen Stelle vorgesehen sein.

Die Welle bzw. der Achszapfen am Lenker 40, der das zweite Gelenk 42 definiert, weist einen größeren Durchmesser auf als das erste Gelenk 41. Ferner ist eine Blattfeder 46 vorgesehen, die mittelbar oder unmittelbar am Prisma 30 befestigt ist und an der Welle bzw. dem Achszapfen am Lenker 40 anliegt. Der größere Durchmesser des zweiten Gelenks 42 und die Blattfeder 46 bewirken, dass im zweiten Gelenk 42 eine deutlich größere Reibung auftritt als im ersten Gelenk 41.

Am Prisma 30 oder mit dem Prisma 30 verbunden ist ein Anschlag 48 vorgesehen, der von dem Prisma 30 in Richtung senkrecht zu den Zeichenebenen der Figuren 4 und 5 zumindest teilweise in die Ebene ragt, in der der Lenker 40 angeordnet ist. Bei den in den Figuren 4 und 5 gezeigten Positionen 31, 33 des Prismas liegt der Anschlag 48 am Lenker 40 an. Dadurch verhindert der Anschlag 48 eine Schwenkbewegung des Prismas 30 relativ zum Lenker 40 im mathematisch positiven Drehsinn bzw. gegen den Uhrzeigersinn (bezogen auf die Darstellungen in den Figuren 4 und 5). Damit verhindert der Anschlag 48 insbesondere eine Bewegung des Prismas 30 über die Position 31 hinaus im mathematisch positiven Sinn bzw. gegen den Uhrzeigersinn und damit auch eine Blickrichtung, die (von der dritten Blickrichtung 25 und der zweiten Blickrichtung 23 ausgehend) über die erste Blickrichtung 21 hinaus geht.

Der Anschlag 48 ermöglicht jedoch eine Drehbewegung des Prismas 30 von der in den Figuren 4 und 5 dargestellten Position 33 im mathematisch negativen Drehsinn bzw. mit dem Uhrzeigersinn zu der in Figur 6 dargestellten Position 35 hin. In der Position 35 des Prismas 30 liegt der Anschlag 48 nicht am Lenker 40 an.

Ferner sind bei dem Ausführungsbeispiel der Figuren 4 bis 6 ein Sperrschieber 62 und ein Eingriffselement 63 vorgesehen. Das Eingriffselement 63 weist insbesondere die Gestalt eines Stifts oder einer Gleitfläche, die sich in Richtung senkrecht zu den Zeichenebenen der Figuren 4 bis 6 erstreckt, auf. Der Sperrschieber 62 ist mit dem Steuerdraht 60 mechanisch gekoppelt, insbesondere gelötet, geklebt oder auf andere Weise gefügt. Zur präzisen Führung des Sperrschiebers 62 entlang eines vorbestimmten geraden Pfads und optional ferner zur Führung des Steuerdrahts in seinem Bereich proximal des Sperrschiebers 62 ist eine Linearführung 68 vorgesehen.

In Figur 4 ist das Prisma 30 in einer ersten Position 31 gezeigt, die der in Figur 1 angedeuteten ersten Blickrichtung 21 entspricht. In Figur 5 ist das Prisma 30 in einer zweiten Position 33 gezeigt, die der zweiten Blickrichtung 23 entspricht. In Figur 6 ist das Prisma 30 in einer dritten Position 35 gezeigt, die der dritten Blickrichtung 25 entspricht. Dabei schlägt gleichzeitig das proximale Ende bzw. die proximale Stirnfläche des Sperrschiebers 62 an einer korrespondierenden Fläche an der Linearführung 68 an, um eine weitere Bewegung des Steuerdrahts 60 und des Sperrschiebers 62 nach proximal zu verhindern. Im Bereich von der ersten Blickrichtung 21 bzw. der ersten Position 31 des Prismas 30 bis zur zweiten Blickrichtung 23 bzw. zur zweiten Position 33 des Prismas 30 ist die Bewegung des Prismas 30 ohne eine Relativbewegung von Prisma 30 und Lenker 40 allein durch ein Schwenken des Lenkers 40 zusammen mit dem Prisma 30 um die durch das erste Gelenk 41 definierte Schwenkachse möglich. Wie erwähnt, ist das erste Gelenk 41 mit dem Vorsprung 44 ausgebildet, um die Schwenkbewegung des Lenkers 40 auf den Bereich zwischen den in den Figuren 4 und 5 gezeigten Positionen bzw. auf den Bereich zwischen der ersten Blickrichtung 21 und der zweiten Blickrichtung 23 zu beschränken. Da, wie erwähnt, die Reibung im zweiten Gelenk 42 größer als im ersten Gelenk 41 ist, findet im Bereich zwischen der ersten Blickrichtung 21 und der zweiten Blickrichtung 23 keine Relativbewegung von Prisma 30 und Lenker 40 statt.

Im Bereich zwischen der zweiten Blickrichtung 23 bzw. der zweiten Position 33 des Prismas 30 und der dritten Blickrichtung 25 bzw. der dritten Position 35 des Prismas 30 sind der Sperrschieber 62 und das Eingriffselement 63 im mechanischen Eingriff bzw. in formschlüssiger Verbindung. Die Begrenzung des Schwenkbereichs des Lenkers 40 durch den Vorsprung 44 am ersten Gelenk 41 einerseits und der Formschluss zwischen dem Sperrschieber 62 und dem Eingriffselement 63 andererseits halten im Bereich zwischen der zweiten Blickrichtung 23 bzw. der zweiten Position 33 des Prismas 30 und der dritten Blickrichtung 25 bzw. der dritten Position 35 des Prismas 30 den Lenker 40 in der in den Figuren 5 und 6 dargestellten Position. In diesem Bereich schwenkt somit das Prisma 30 ausschließlich um die durch das zweite Gelenk 42 definierte Schwenkachse.

Anstelle des Vorsprungs 44 am ersten Gelenk 41 zur Begrenzung der Bewegung im ersten Gelenk 41 und anstelle des Anschlags 48 zur Begrenzung der Bewegung im zweiten Gelenk 42 können andere mechanische Einrichtungen vorgesehen sein, die den Schwenkbereich des Lenkers 40 relativ zum distalen Ende 12 des Endoskops 10 bzw. den Schwenkbereich des Prismas 30 relativ zum Lenker 40 begrenzen.

Insgesamt zerfällt somit aufgrund der Beschränkung der Schwenkbewegung am ersten Gelenk 41, der unterschiedlichen Reibungen im ersten Gelenk 41 und im zweiten Gelenk 42 und des von der Position des Steuerdrahts 60 abhängigen Eingriffs zwischen dem Sperrschieber 62 und dem Eingriffselement 63 der Winkelbereich 20 von Blickrichtungen 21, 23, 25 (vgl. Figur 1) in einen ersten Teilbereich 22 und einen zweiten Teilbereich 24. Im ersten Teilbereich 22 zwischen der ersten Blickrichtung 21 und der zweiten Blickrichtung 23 schwenkt das Prisma 30 ausschließlich um die durch das erste Gelenk 41 definierte Schwenkachse, und der Anschlag 48 liegt am Lenker 40 an. Im zweiten Teilbereich 24 zwischen der zweiten Blickrichtung 23 und der dritten Blickrichtung 25 schwenkt das Prisma 30 ausschließlich um die durch das zweite Gelenk 42 definierte Schwenkachse.

Ähnliche Ergebnisse können ohne Sperrschieber 62, Eingriffselement 63 und unterschiedliche Reibung in den Gelenken 41, 42 beispielsweise erzielt werden, indem mittels einer Feder oder eines elastischen Elements der Lenker 40 in die in den Figuren 5 und 6 gezeigte Position vorgespannt wird, und/oder indem durch eine Feder oder ein anderes elastisches Element das Prisma 30 und der Lenker 40 in die in den Figuren 4 und 5 gezeigte relative Position vorgespannt werden.

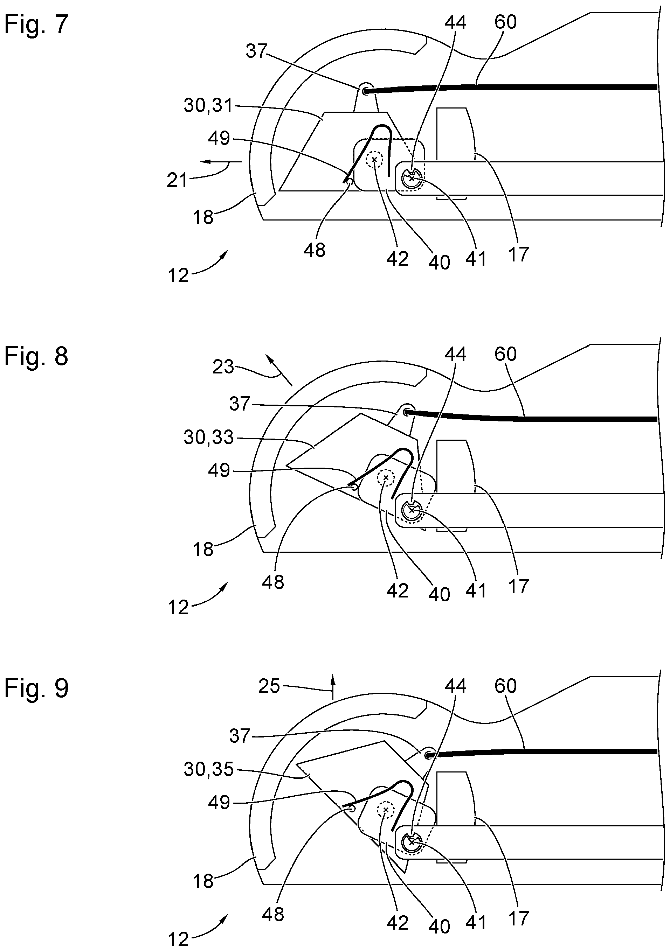

Die Figuren 7 bis 9 zeigen ein Ausführungsbeispiel eines distalen Endes 12 eines Endoskops, das in einigen Merkmalen dem Ausführungsbeispiel der Figuren 4 bis 6 ähnelt, insbesondere in der Ausbildung der Lichtlenkeinrichtung 30 als Prisma, in der Begrenzung des für das Prisma 30 zur Verfügung stehenden Bauraums durch eine distale Linse 17 und ein Fensterbauteil 18, in der Lagerung des Prismas 30 mittels eines Lenkers 40 im Schaft 13 des Endoskops sowie in der mechanischen Steuerung des Prismas 30 mittels eines Steuerdrahts 60, dessen distales Ende gelenkig mit einem Steuerhebel 37 am Prisma 30 gekoppelt ist.

Im Unterschied zu dem Ausführungsbeispiel der Figuren 4 bis 6 ist beim Ausführungsbeispiel der Figuren 7 bis 9 eine Blattfeder 49 vorgesehen, deren eines (in den Figuren 7 bis 9 rechtes) Ende starr mit dem Lenker 40, und deren anderes (in den Figuren 7 bis 9 linkes) Ende am Anschlag 48 anliegt. Die Blattfeder 49 drückt den Anschlag 48 elastisch gegen den Lenker 40. Damit spannt die Blattfeder 49 das Prisma 30 in Richtung zu der in den Figuren 7 und 8 dargestellten Position relativ zum Lenker 40 vor.

Die Blattfeder 49 bewirkt, dass ausgehend von der in Figur 7 dargestellten ersten Blickrichtung 21 und der ersten Position 31 des Prismas 30 bei Bewegung des Steuerdrahts 60 nach proximal der Anschlag 48 zunächst weiterhin am Lenker 40 anliegt und das Prisma 30 zusammen mit dem Lenker 40 gemeinsam um die durch das erste Gelenk 41 definierte Schwenkachse geschwenkt wird. Bei der in Figur 8 dargestellten zweiten Blickrichtung 23 und der zweiten Position 33 des Prismas 30 hat der Lenker 40 seine durch den Vorsprung 44 am ersten Gelenk 41 definierte maximale Auslenkung erreicht. Wenn der Steuerdraht 60 weiter nach proximal gezogen wird, muss deshalb das Prisma 30 relativ zum Lenker 40 geschwenkt werden, wobei der Anschlag 48 vom Lenker 40 abgehoben wird. Deshalb geschieht die Bewegung des Prismas 30 von der zweiten Position 33 zur in Figur 9 dargestellten dritten Position 35 gegen die elastische Kraft der Blattfeder 49.

Wenn ausgehend von der in Figur 9 dargestellten dritten Position 35 des Prismas 30 der Steuerdraht 60 nach distal bewegt wird, drückt die Blattfeder 49 den Anschlag 48 zum Lenker 40 hin. Deshalb findet zunächst eine Schwenkbewegung des Prismas 30 um die durch das zweite Gelenk 42 definierte Schwenkachse relativ zum Lenker 40 statt, wobei der Lenker 40 unbewegt bleibt. Erst bei der in Figur 8 dargestellten zweiten Position 33 des Prismas 30 liegt der Anschlag 48 am Lenker 40 an. Eine weitere Bewegung des Prismas 30 relativ zum Lenker 40 ist dann nicht mehr möglich. Deshalb schwenkt bei einer weiteren Bewegung des Steuerdrahts 60 nach distal das Prisma 30 gemeinsam mit dem Lenker 40 um die durch das erste Gelenk 41 definierte Schwenkachse.

Bei dem anhand der Figuren 7 bis 9 dargestellten Ausführungsbeispiel können einige Merkmale des Ausführungsbeispiels der Figuren 4 bis 6 entfallen, insbesondere der Sperrschieber 62, die Blattfeder 46 am zweiten Gelenk 42 und der vergrößerte Durchmesser am zweiten Gelenk 42.

Bei dem anhand der Figuren 7 bis 9 dargestellten Ausführungsbeispiel kann anstelle der Blattfeder 49 ein anderes elastisches Element vorgesehen sein, das das Prisma 30 in die in den Figuren 7 und 8 dargestellte Position relativ zum Lenker 40 vorspannt. Alternativ oder zusätzlich kann eine weitere Blattfeder oder ein weiteres elastisches Element vorgesehen sein, das den Lenker in Richtung zu der in den Figuren 8 und 9 dargestellten Position relativ zum distalen Ende 12 des Endoskops vorspannt. Ferner kann alternativ ein einziges elastisches Element vorgesehen sein, das sowohl das Prisma 30 in Richtung zu der in den Figuren 7 und 8 dargestellten Position relativ zum Lenker 40 als auch den Lenker 40 in Richtung zu der in den Figuren 8 und 9 dargestellten Position relativ zum distalen Ende 12 des Endoskops vorspannt.

Anstelle der anhand der Figuren 4 bis 9 dargestellten Maßnahmen zur Festlegung, in welchen Winkelbereichen das Prisma um die durch die Gelenke 41, 42 definierten Schwenkachsen geschwenkt wird, oder zusätzlich zu diesen sind auch andere Maßnahmen möglich. Beispielsweise können am Sperrschieber 62 und am Lenker 40 an Oberflächenbereichen, die parallel zu einander sind und die einander berühren, gerade oder gekrümmte feine Rillen oder andere Texturen vorgesehen sein, um die Schwenkbewegung des Lenkers 40 durch die Linearbewegung des Sperrschiebers 62 zu unterstützen.

Ferner können an dieser oder an anderen Stellen Reibbeläge vorgesehen sein. Beispielsweise können an einer zu den Zeichenebenen der Figuren 4 bis 9 parallelen Fläche am ersten Gelenk 41 und an einem mit dem Sperrschieber 62 linear verschiebbaren Element Reibbeläge vorgesehen sein. Diese Reibbeläge können so angeordnet und ausgebildet sein, dass sie nur in einem vorbestimmten Winkelbereich, insbesondere nur im Bereich zwischen der zweiten Position 33 und der dritten Position 35 des Prismas in Eingriff sind und ein Schwenken des Lenkers 40 um die durch das erste Gelenk 41 definierte Schwenkachse hemmen.

Alternativ oder zusätzlich zu Reibbelägen und/oder formschlüssiger Steuerung mittels eines oder mehrerer Steuerschieber sind Magnete verwendbar, um eine Schwenkbewegung um eine Schwenkachse zu fördern oder zu hemmen. Magnete können auch anstelle von Federn oder anderen elastischen Elementen verwendbar sein. Eine Hemmung ist auch mittels eines oder mehrerer Piezoelemente möglich.

Zum Antrieb des Prismas 30 können anstelle des Steuerdrahts oder zusätzlich zu diesem weitere oder andere Mittel vorgesehen sein. Beispielsweise kann der Lenker 40 unabhängig von dem Prisma 30 mittels eines weiteren Steuerdrahts bewegt werden. Zwei an gegenüberliegenden Seiten des Prismas 30 parallel zueinander angeordnete Lenker 40 können mittels zweier Steuerdrähte bewegt werden. Alle zwei, drei oder mehr Steuerdrähte sind insbesondere am proximalen Ende 11 des Endoskops 10 miteinander gekoppelt.

Anstelle eines oder mehrerer Steuerdrähte können ein oder mehrere elektromotorische, magnetische, pneumatische, hydraulische, piezomotorische oder sonstige Antriebe vorgesehen sein. Ferner sind Antriebe unter Verwendung von Bimetallfedern oder anderen Anordnungen, die sich aufgrund unterschiedlicher thermischer Ausdehnungskoeffizienten temperaturabhängig verformen, verwendbar. Ferner sind Antriebe unter Verwendung von Formgedächtnislegierungen (engl.: shape memory alloys) verwendbar, die sich eine Formumwandlung aufgrund einer temperaturabhängigen Gitterumwandlung zunutze machen.

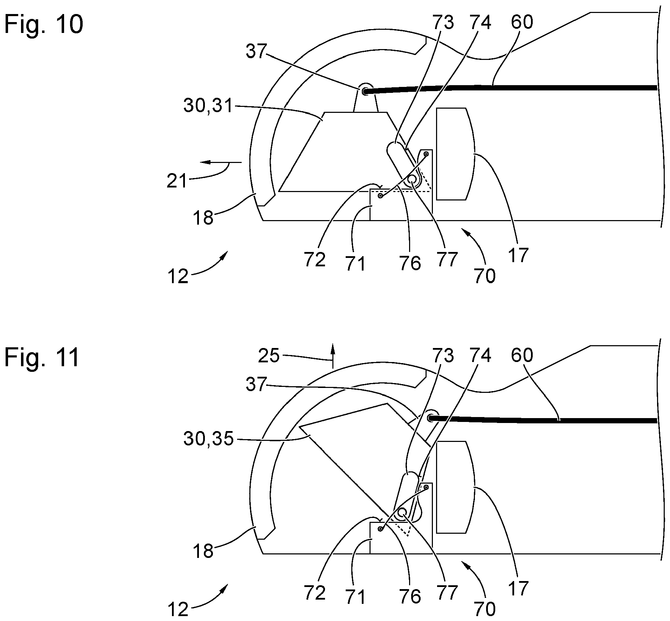

Die Figuren 10 und 11 zeigen schematische Darstellungen eines weiteren Ausführungsbeispiels eines distalen Endes 12 eines Endoskops, das in einigen Merkmalen den Ausführungsbeispielen der Figuren 2 bis 6 ähnelt. Insbesondere ist ein Prisma 30 als Lichtlenkeinrichtung zwischen einem Fensterbauteil 18 und einer distalen Linse 17 vorgesehen, das mittels eines Steuerdrahts 60 gesteuert bzw. bewegt werden kann.

Anders als bei den Ausführungsbeispielen der Figuren 2 bis 9 ist das Prisma 30 nicht über einen oder mehrere Lenker im Endoskop gelagert. Stattdessen ist eine Gleitflächeneinrichtung 70 vorgesehen. Die Gleitflächeneinrichtung 70 umfasst einen feststehenden Gleitflächenkörper 71 mit einer Gleitfläche 72 und einen Gleitflächenkörper 73 am Prisma 30 mit einer Gleitfläche 74. Die Gleitfläche 72 am feststehenden Gleitflächenkörper 71 ist im Wesentlichen L-förmig. Der Gleitflächenkörper am Prisma 30 ist länglich bzw. stabförmig. Insbesondere das dem feststehenden Gleitflächenkörper 71 zugewandte Ende des Gleitflächenkörpers 73 am Prisma 30 ist abgerundet. Ferner umfasst die Gleitflächeneinrichtung 70 eine Blattfeder 76, deren Enden am feststehenden Gleitflächenkörper 71 gelagert sind, und einen Stift 77 am Gleitflächenkörper 73 am Prisma 30. Die Blattfeder 76 liegt am Stift 77 an und drückt die Gleitfläche 74 am Gleitflächenkörper am Prisma 30 gegen die Gleitfläche 72 am feststehenden Gleitflächenkörper 71.

Die Gleitflächen 72, 74, die Blattfeder 76 und der Stift 77 sind so ausgebildet und angeordnet, dass das Prisma 30 bei jeder möglichen Orientierung eine wohl definierte vorbestimmte Position einnimmt. Dabei liegt die Gleitfläche 74 am Gleitflächenkörper 73 am Prisma 30 in einem zusammenhängenden Bereich (beispielsweise die in Figur 10 angedeutete Situation) oder in zwei räumlich getrennten Bereichen (beispielsweise in Figur 8 angedeutete Situation) an der Gleitfläche 72 am feststehenden Gleitflächenkörper 71 an. Durch die Gestalt und Anordnung der Gleitfläche 72 am feststehenden Gleitflächenkörper 71 und die Gestalt und Anordnung der Gleitfläche 74 am Gleitflächenkörper 73 am Prisma 30 kann die Bewegung zwischen einer in Figur 1 dargestellten ersten Position 31 des Prismas 30, die der ersten Blickrichtung 21 entspricht, und einer dritten Position 35, die der dritten Blickrichtung 25 entspricht, nahezu beliebig eingestellt bzw. bestimmt werden.

Bei jedem der Ausführungsbeispiele der Figuren 2 bis 8 kann jeweils eine andere Lichtlenkeinrichtung anstelle des Prismas 30 vorgesehen sein, beispielsweise ein Objektiv, eine Linse oder ein Spiegel. Insbesondere wenn die Lichtlenkeinrichtung ein Objektiv ist oder ein Objektiv umfasst, kann ein lichtempfindlicher Bildsensor mit dem Objektiv starr gekoppelt sein. Ferner können mehrere Lichtlenkeinrichtungen vorgesehen sein, die unterschiedliche Bewegungen ausführen können.

### Bezugszeichen

- 10: Endoskop
- 11: proximales Ende des Endoskops 10
- 12: distales Ende des Endoskops 10
- 13: Schaft des Endoskops 10
- 14: Okular des Endoskops 10
- 15: Kupplung am Endoskop 10
- 17: distale Linse im Beobachtungsstrahlengang
- 18: Fensterbauteil im Beobachtungsstrahlengang
- 20: Winkelbereich von Blickrichtungen
- 21: erste Blickrichtung
- 22: erster Teilbereich
- 23: zweite Blickrichtung
- 24: zweiter Teilbereich
- 25: dritte Blickrichtung
- 30: Prisma (Lichtlenkeinrichtung)
- 31: erste Position des Prismas 30
- 33: zweite Position des Prismas 30
- 35: dritte Position des Prismas 30
- 37: Steuerhebel am Prisma 30
- 40: (erster) Lenker
- 41: erstes Gelenk
- 42: zweites Gelenk
- 44: Vorsprung am ersten Gelenk 41
- 46: Blattfeder am zweiten Gelenk 42
- 48: Anschlag für das zweite Gelenk 42
- 49: Blattfeder am zweiten Gelenk 42
- 50: zweiter Lenker
- 51: drittes Gelenk
- 52: viertes Gelenk
- 60: Steuerdraht
- 62: Sperrschieber
- 63: Eingriffselement am Prisma 30 für Sperrschieber 62
- 68: Linearführung für Steuerdraht 60 und Sperrschieber 62
- 70: Gleitflächeneinrichtung
- 71: feststehender Gleitflächenkörper
- 72: Gleitfläche am feststehenden Gleitflächenkörper 71
- 73: Gleitflächenkörper am Prisma 30
- 74: Gleitfläche am Gleitflächenkörper 73 am Prisma 30
- 76: Blattfeder
- 77: Stift

## Patentansprüche

1. Endoskop (10) mit einer Blickrichtung (21, 23, 25), die innerhalb eines vorbestimmten Winkelbereichs (20) einstellbar ist, mit:
einer bewegbaren Lichtlenkeinrichtung (30) am distalen Ende (12) des Endoskops (10), von deren Orientierung die Blickrichtung (21, 23, 25) des Endoskops (10) abhängt,
einer Gelenkeinrichtung (40, 50; 70) zum bewegbaren Halten der Lichtlenkeinrichtung (30),
wobei die Gelenkeinrichtung (40, 50; 70) für eine Bewegbarkeit der Lichtlenkeinrichtung (30) ausgebildet ist, die mehr als nur eine Schwenkbarkeit um eine einzige Schwenkachse umfasst,
wobei die Bewegbarkeit der Lichtlenkeinrichtung (30) eine Abfolge von Schwenkbewegungen um verschiedene Schwenkachsen umfasst, die parallel zueinander sind, wobei die Gelenkeinrichtung (40) ein erstes Gelenk (41), das eine erste Schwenkachse definiert, und ein zweites Gelenk (42), das eine zweite Schwenkachse definiert, umfasst
**dadurch gekennzeichnet, dass** die Lichtlenkeinrichtung (30) zumindest entweder ein Prisma oder einen Spiegel umfasst.

2. Endoskop (10) nach dem vorangehende Anspruch, wobei
das erste Gelenk (41) ausgebildet und angeordnet ist, um eine Schwenkbewegung der Lichtlenkeinrichtung (30) um die erste Schwenkachse zu ermöglichen, wenn die Blickrichtung (21, 23, 25) in einem ersten Teilbereich (22) des vorbestimmten Winkelbereichs (20) liegt, und das zweite Gelenk (42) ausgebildet und angeordnet ist, um eine Schwenkbewegung der Lichtlenkeinrichtung (30) um die zweite Schwenkachse zu ermöglichen, wenn die Blickrichtung (21, 23, 25) in einem zweiten Teilbereich (24) des vorbestimmten Winkelbereichs (20) liegt.

3. Endoskop (10) nach dem vorangehenden Anspruch, bei dem der erste Teilbereich (22) und der zweite Teilbereich (24) aneinander angrenzen.

4. Endoskop (10) nach Anspruch 2 oder 3, ferner mit:
einer Einrichtung (46) zum Hemmen einer Schwenkbewegung um die zweite Schwenkachse, wobei die Einrichtung (46) zum Hemmen ausgebildet ist, um die Schwenkbewegung zumindest dann zu hemmen, wenn die Blickrichtung (21, 23, 25) außerhalb des zweiten Teilbereichs (24) liegt.

5. Endoskop (10) nach einem der Ansprüche 2 bis 4, ferner mit:
einer Einrichtung (44, 62, 63) zum Blockieren einer Schwenkbewegung um die erste Schwenkachse, wobei die Einrichtung (44, 62, 63) zum Blockieren ausgebildet ist, um die Schwenkbewegung um die erste Schwenkachse zu blockieren, wenn die Blickrichtung (21, 23, 25) nicht innerhalb des ersten Teilbereichs (22) liegt.

6. Endoskop (10) nach einem der Ansprüche 1 bis 4, ferner mit:
einem elastischen Element (49), das so mit der Gelenkeinrichtung (40, 50) gekoppelt ist, dass zumindest entweder das erste Gelenk (41) in Richtung zu einer ersten vorbestimmten Winkelposition vorgespannt ist oder das zweite Gelenk (42) in Richtung zu einer zweiten vorbestimmten Winkelposition vorgespannt ist.

7. Endoskop (10) nach Anspruch 1, bei dem die Gelenkeinrichtung einen ersten Lenker (40), der das erste Gelenk (41) und das zweite Gelenk (42) verbindet, und einen zweiten Lenker (50), der ein drittes Gelenk (51) und ein viertes Gelenk (52) verbindet, umfasst, wobei das zweite Gelenk (42) und das vierte Gelenk (52) mit der Lichtlenkeinrichtung (30) mechanisch starr verbunden sind.

8. Endoskop (10) nach dem vorangehenden Anspruch, bei dem die Gelenkeinrichtung zumindest entweder einen Lenker oder eine Nockensteuerung oder ein elastisches Element (76) umfasst.

9. Endoskop (10) nach einem der vorangehenden Ansprüche, ferner mit einem lichtempfindlichen Bildsensor, wobei der lichtempfindliche Bildsensor so mit der Lichtlenkeinrichtung (30) gekoppelt ist, dass er mit der Lichtlenkeinrichtung (30) bewegbar ist.

## Claims

1. Endoscope (10) with a direction of view (21, 23, 25) which is adjustable within a predetermined angular range (20), comprising:
a movable light-guiding device (30) at the distal end (12) of the endoscope (10), the direction of view (21, 23, 25) of the endoscope (10) depending on the orientation thereof,
a hinge device (40, 50; 70) for movably holding the light-guiding device (30),
wherein the hinge device (40, 50; 70) is embodied for a movability of the light-guiding device (30), which comprises more than only one pivotability about a single pivot axis, wherein the movability of the light-guiding device (30) comprises a sequence of pivot movements about different pivot axes which are parallel to one another, wherein the hinge device (40) comprises a first hinge (41), which defines a first pivot axis, and a second hinge (42), which defines a second pivot axis,
**characterized in that** the light-guiding device (30) comprises at least either a prism or a mirror.

2. Endoscope (10) according to the preceding claim, wherein
the first hinge (41) is embodied and arranged to facilitate a pivot movement of the light-guiding device (30) about the first pivot axis if the direction of view (21, 23, 25) lies in a first portion (22) of the predetermined angular range (20) and
the second hinge (42) is embodied and arranged to facilitate a pivot movement of the light-guiding device (30) about the second pivot axis if the direction of view (21, 23, 25) lies in a second portion (24) of the predetermined angular range (20).

3. Endoscope (10) according to the preceding claim, in which the first portion (22) and the second portion (24) adjoin one another.

4. Endoscope (10) according to Claim 2 or 3, further comprising:
a device (46) for inhibiting a pivot movement about the second pivot axis, wherein the device (46) for inhibiting is embodied to inhibit the pivot movement at least when the direction of view (21, 23, 25) lies outside of the second portion (24).

5. Endoscope (10) according to any one of Claims 2 to 4, further comprising:
a device (44, 62, 63) for blocking a pivot movement about the first pivot axis, wherein the device (44, 62, 63) for blocking is embodied to block the pivot movement about the first pivot axis when the direction of view (21, 23, 25) does not lie within the first portion (22).

6. Endoscope (10) according to any one of Claims 1 to 4, further comprising:
an elastic element (49) which is coupled to the hinge device (40, 50) in such a way that at least either the first hinge (41) is pretensioned in the direction of a first predetermined angle position or the second hinge (42) is pretensioned in the direction of a second predetermined angle position.

7. Endoscope (10) according to Claim 1, wherein the hinge device comprises a first rod (40) which connects the first hinge (41) and the second hinge (42) and a second rod (50) which connects a third hinge (51) and a fourth hinge (52), wherein the second hinge (42) and the fourth hinge (52) are connected to the light-guiding device (30) in a mechanically rigid manner.

8. Endoscope (10) according to the preceding claim, wherein the hinge device comprises at least either a rod or a cam control or an elastic element (76).

9. Endoscope (10) according to any one of the preceding claims, further comprising a light-sensitive image sensor, wherein the light-sensitive image sensor is coupled to the light-guiding device (30) in such a way that it is movable together with the light-guiding device (30).

## Revendications

1. Endoscope (10) ayant une direction de visée (21, 23, 25) pouvant être réglée à l'intérieur d'un domaine angulaire prédéterminé (20), comportant :
un dispositif de déviation de lumière mobile (30) à l'extrémité distale (12) de l'endoscope (10), la direction de visée (21, 23, 25) de l'endoscope (10) dépendant de l'orientation dudit dispositif,
un dispositif d'articulation (40, 50 ; 70) destiné à maintenir de manière mobile le dispositif de déviation de lumière (30),
dans lequel le dispositif d'articulation (40, 50 ; 70) est réalisé de manière à permettre une mobilité du dispositif de déviation de lumière (30), qui comprend plus de seulement une possibilité de pivotement autour d'un axe de pivotement unique,
dans lequel la mobilité du dispositif de déviation de lumière (30) comprend une succession de mouvements de pivotement autour d'axes de pivotement différents qui sont parallèles les uns aux autres,
dans lequel le dispositif d'articulation (40) comprend une première articulation (41) qui définit un premier axe de pivotement et une deuxième articulation (42) qui définit un deuxième axe de pivotement,
**caractérisé en ce que** le dispositif de déviation de lumière (30) comprend au moins soit un prisme soit un miroir.

2. Endoscope (10) selon la revendication précédente, dans lequel
la première articulation (41) est réalisée et disposée de manière à permettre un mouvement de pivotement du dispositif de déviation de lumière (30) autour du premier axe de pivotement lorsque la direction de visée (21, 23, 25) se situe dans un premier domaine partiel (22) du domaine angulaire prédéterminé (20), et
la deuxième articulation (42) est réalisée et disposée de manière à permettre un mouvement de pivotement du dispositif de déviation de lumière (30) autour du deuxième axe de pivotement lorsque la direction de visée (21, 23, 25) se situe dans un deuxième domaine partiel (24) du domaine angulaire prédéterminé (20).

3. Endoscope (10) selon la revendication précédente, dans lequel le premier domaine partiel (22) et le deuxième domaine partiel (24) sont adjacents l'un à l'autre.

4. Endoscope (10) selon la revendication 2 ou 3, comportant en outre :
un dispositif (46) d'inhibition d'un mouvement de pivotement autour du deuxième axe de pivotement, dans lequel le dispositif (46) d'inhibition est réalisé pour au moins n'inhiber le mouvement de pivotement que lorsque la direction de visée (21, 23, 25) se situe à l'extérieur du deuxième domaine partiel (24).

5. Endoscope (10) selon l'une des revendications 2 à 4, comportant en outre :
un dispositif (44, 62, 63) destiné à bloquer un mouvement de pivotement autour du premier axe de pivotement, dans lequel le dispositif (44, 62, 63) de blocage est réalisé de manière à bloquer le mouvement de pivotement autour du premier axe de pivotement lorsque la direction de visée (21, 23, 25) ne se situe pas à l'intérieur du premier domaine partiel (22).

6. Endoscope (10) selon l'une quelconque des revendications 1 à 4, comportant en outre :
un élément élastique (49) qui est couplé au dispositif d'articulation (40, 50) soit de manière à ce que la première articulation (41) soit précontrainte dans une direction orientée vers une première position angulaire prédéterminée, soit de manière à ce que la deuxième articulation (42) soit précontrainte dans une direction orientée vers une deuxième position angulaire prédéterminée.

7. Endoscope (10) selon la revendication 1, dans lequel le dispositif d'articulation comprend une première bielle (40) qui relie la première articulation (41) à la deuxième articulation (42), et une deuxième bielle (50) qui relie une troisième articulation (51) à une quatrième articulation (52), dans lequel la deuxième articulation (42) et la quatrième articulation (52) sont reliées mécaniquement de manière rigide au dispositif de déviation de lumière (30).

8. Endoscope (10) selon la revendication précédente, dans lequel le dispositif d'articulation comprend au moins soit une bielle, soit un dispositif de commande à cames, soit un élément élastique (76).

9. Endoscope (10) selon l'une des revendications précédentes, comportant en outre un capteur d'image sensible à la lumière, dans lequel le capteur d'image sensible à la lumière est couplé au dispositif de déviation de lumière (30) de manière à ce qu'il puisse être déplacé avec le dispositif de déviation de lumière (30).
